# EUROPEAN PATENT APPLICATION

(11) **EP 1 859 755 A2**
(43) Date of publication of application: **28.11.2007**
(21) Application number: 07108702.7
(22) Date of filing: 22.05.2007
(51) Int. Cl.: A61B 19/00

(54) **Method and system for computer-assisted femoral head resurfacing**

(30) Priority: 22.05.2006 GB 0610079
(71) Applicant: Finsbury (Development) Limited, Leatherhead, Surrey KT22 0BA (GB)
(72) Inventor: Tuke, Michael Antony, Guildford, Surrey GU1 3TF (GB)
(74) Representative: Smaggasgale, Gillian Helen

(57) **Abstract**

A system for use by a surgeon during implantation of a femoral head resurfacing prosthesis in a patient which will locate the correct position for the femoral head resurfacing prosthesis, the system comprising:
(a) a computer having memory for holding data relating to the size and shape of at least one femoral head resurfacing implant, and data obtained during preoperative scanning of the patient's hip joint;
(b) said computer having a display suitable for displaying a model of the patient's hip joint; and
(c) said computer being programmed to:
(i) determine the pre-operative head center position of the patient's femoral head;
(ii) optionally amend the determined head center position;
(iii) overlay the data relating to the size and shape of the at least one femoral implant on the model;
(iv) simulate the range of motion obtainable with the overlaid prosthetic implant;
(v) repeat steps (iii) and (iv) as required optionally using a different size hip resurfacing prosthetic component at chosen head center position to select optimum femoral implant;
(vi) simulate joining the selected prosthetic component to the bony neck; and optionally
(vii) determine whether the size is correct and the angle for the orientation of the cylindrical axis relative to the femoral bone.

## Description

The present invention relates to a system for use by a surgeon during implantation of a femoral head resurfacing prosthesisin a patient. In alternative aspects, the present invention relates to a computer element, a computer program and a media including the computer program for use in femoral head resurfacing operations. In a still further aspect of the present invention there is provided a computer programmed with a computer program, for use in femoral head resurfacing operations. In an even further aspect, the present invention relates to a method for locating the correct position for a hip resurfacing prosthesis.

The efficient functioning of the hip joint is extremely important to the well being and mobility of the human body. Each hip joint is comprised by the upper portion of the femur which terminates in an offset bony neck surmounted by a ball-headed portion which rotates within the acetabulum in the pelvis. Diseases such as rheumatoid- and osteo-arthritis can cause erosion of the cartilage lining of the acetabulum so that the ball of the femur and the hip bone rub together causing pain and further erosion. Bone erosion may cause the bones themselves to attempt to compensate for the erosion which may result in the bone being reshaped. This misshapen joint may cause pain and may eventually cease to function altogether.

Operations to replace the hip joint with an artificial implant are well-known and widely practiced. Generally, the hip prosthesis will be formed of two components, namely: an acetabular, or socket, component which lines the acetabulum; and a femoral, or stem, component which replaces the femoral head. During the surgical procedure for implanting the hip prosthesis the cartilage is removed from the acetabulum using a reamer such that it will fit the outer surface of the acetabular component of the hip prosthesis. The acetabular component can then be inserted into place. In some arrangements, the acetabular component may simply be held in place by a tight fit with the bone. However, in other arrangements, additional fixing means such as screws or bone cement may be used. The use of additional fixing means help to provide stability in the early stages after the prosthesis has been inserted. In some modem prosthesis, the acetabular component may be coated on its external surface with a bone growth promoting substance which will assist the bone to grow and thereby assist the holding of the acetabular component in place. The bone femoral head will be removed and the femur hollowed using reamers and rasps to accept the prosthesis. The stem portion will then be inserted into the femur.

However, it is now becoming more increasingly common for the femoral component of the kind described above to be replaced with components for use in femoral head resurfacing.

In femoral head resurfacing techniques, the surgeon shapes the head of the femur to fit within the cavity of the resurfacing prosthesis.

It will be acknowledged that it is essential that the replacement surface for the head of the femur should be precisely located in both angular and translation positions of the axis of the femoral neck. To assist this, in some techniques, the surgeon inserts a pin in the lateral femur. The desired position of the pin is assessed from pre-operative analysis of the x-rays or other scans. The surgeon will measure the desired distance down the femur from the tip of the greater trochanter and the alignment pin is inserted through the vastus lateralis fibres. The alignment pin is inserted in a transverse direction into the mid-lateral cortex and directed upwardly towards the femoral head. The pin is left protruding so that an alignment guide can be hooked over the alignment pin. Suitable alignment guides include those known as the McMinn Alignment Guide available from Midland Medical Technologies Ltd.

These alignment guides generally comprise a hook or aperture which is placed over the alignment pin thus providing a good angular position for the axis of the implant in valgus, varus and ante-version of the neck. The guide will then be adjusted so that a cannulated rod is located such that the aperture therein is directed down the mid-lateral axis of the femoral neck. A stylus having been set to the desired femoral component size is positioned such that it can be passed around the femoral neck. When the stylus can be passed around the femoral neck, the cannulated rod is locked in position. Once the guide is stabilised in this way fine adjustments can be made until the surgeon is happy that the guide is in the required position.

A guide wire can then be inserted though the cannulated rod. This guide wire is then used in the further surgery in which the femoral head is shaped to accept the prosthesis. It will be understood that the alignment guide is an essential tool in the surgical procedure to ensure that the aperture drilled in the femoral head is both central to the femoral neck and at the correct angle of alignment to the femoral neck and that the shaping of the femoral head is accurate for the chosen head size.

It will therefore be understood that it is very important that the alignment guide is positioned correctly. Failure to do so may have the disastrous effect of allowing the machining of the cylinder of the head during the shaping procedure to "notch" into the neck of the femur. This will predispose the bone to early failure on load bearing.

In a move to less invasive surgery in which the required incision should be as small as possible and the amount of interaction with healthy tissue is minimised, various tools have been suggested which enable the resurfacing procedure to be carried out without the need to insert the alignment pin i.e. where all of the surgical procedure takes place at the femoral head. Various alignment guides have been suggested which interact with the femoral head and/or neck. Tools for checking the correct alignment have also been suggested. Examples of these prior art tools are described in European patent publications EP1588668, EP 1588669, EP 1634550, EP 1776937 and European patent application 06270098.4 filed 8th December 2006.

Other guides are known which are, in use, located on the femoral neck itself. These are used in a similar manner to those described above and may involve some adjustment by the surgeon before he selects the best position.

As indicated above, femoral head resurfacing techniques require the surgeon to shape the head of the femur to fit within the cavity of the resurfacing prosthesis. This generally involves a number of shaping steps including the removal of the dome of the femoral head by means of a saw. It is important that the saw cut is made in the correct position so that an accurate fit with the prosthesis can be achieved. The position of the cut to remove the dome of the femoral head is generally calculated from the top of the dome of the femoral head.

During the surgery, a well is generally drilled into the head of the femur. Cylinder cutters can then be used and kept in alignment by means of a peg placed in the well. These cutters are arranged such that the diameter of the bone of the femoral head after machining will be correct for the size of femoral head prosthesis chosen. In addition, the cutters are selected such that when cutting they will not extend beyond the bottom of the machined femoral head such that the teeth of the cutter do not dangerously over-sail the head-neck junction and cause soft tissue damage or neck notching. The cut head may then be chamfered.

Whilst hip resurfacing has become an accepted method of hip replacement surgery and excellent results have been obtained particularly for younger more active patients where return to normal activity levels and range of motion can be expected, it will be understood that the placement of the two components is critical particularly if wear of parts and impingement of the retained natural bony femoral neck on the edge of the cup or the natural acetabulum rim is to be minimised and preferably avoided.

The prior art arrangements for assisting in the correct positioning of the femoral head work from the long accepted premise of centering on the bony neck and achieving an angulation with relation to the bone of the femur distal to the head. This assists in preventing notching by cutters and prevents too much varus being obtained which would increase the lever arm length. Both notching and an increased lever arm length will result in higher risk of failure of the prosthetic joint due to fracture of the cantilevered femoral neck.

To date, the method for positioning the head on the femur, which it is acknowledged is of paramount importance, has worked from first deciding on an angle for the head axis in both valgus-varus and ante-retro version and then to center on the neck. Finally, the proximal-distal position of the head has been a somewhat arbitrary choice resulting from positioning the edge of the opening (also known as the edge of the skirt) of the ball component of the head resurfacing prosthesis at the junction of the cylinder cut on the bone.

Whilst this established and accepted technique for positioning the head has achieved satisfactory results to date, there is a need for an improved procedure which will enable the prosthesis to be more accurately sized and positioned to provide the patient with optimum expectancy as to recovery and life expectancy of prosthesis.

The present invention relates to altering the accepted procedure for deciding on the position of the prosthesis and to find the angle of the head axis as the last in a sequence of events instead of the conventional process of deciding this first. In contrast, the present invention is based on the finding that a more accurate determination of the position can be obtained by determining the correct position of the head center.

It will help in the understanding of the invention to remember that all loading through a ball joint is passed through the center of the ball.

According to the present invention there is provided a system for use by a surgeon during implantation of a femoral head resurfacing prosthesis in a patient which will locate the correct position for the femoral head resurfacing prosthesis, the system comprising:
(a) a computer having memory for holding data relating to the size and shape of at least one femoral head resurfacing prosthesis, and data obtained during preoperative scanning of the patient's hip joint;
(b) said computer having a display for displaying a model of the patient's hip joint; and
(c) said computer being programmed to:
   (i) determine the pre-operative head center position of the patient's femoral head;
   (ii) optionally amend the determined head center position;
   (iii) overlay the data relating to the size and shape of the at least one femoral head resurfacing prosthesis on the model;
   (iv) simulate the range of motion obtainable with the overlaid prosthetic implant;
   (v) repeat steps (iii) and (iv) as required optionally using a different size of hip resurfacing prosthesis at the chosen head center position to select the optimum femoral head resurfacing prosthesis;
   (vi) simulate joining the selected prosthetic component to the bony neck; and
   (vii) optionally determine whether the size is correct and the angle for the orientation of the cylindrical axis relative to the femoral bone.

The computer memory preferably holds the data as described in (a) above.

The data required to produce the model of the patient's hip can be input into the computer memory by any suitable means. In one arrangement, scanning techniques such as X-Ray, computer aided tomography scanning and the like may be used to provide the dimensions to form the model. The provision of the data, which includes dimensions and the like may be automatic from the scanning procedure or may require measurement and/or calculation and for the data to be input into the computer memory.

In one arrangement a number of landmark points of some critical elements of the bone surface may be plotted at rest and while the patient's leg is moving. This will generally be done at an early stage of joint exposure but prior to the dislocation of the hip joint. Any suitable arrangement of recording the position of these landmark points may be used. Examples of suitable techniques are described in EP1226788. For example, marker means may be attachment to appropriate positions on the bones at and/or near the joint. Pointer means may be provided to touch significant features on the joint. The marker means and the pointer means may all be adapted for operative coupling to a detector means. In a system of this type, the detector means may comprise an infra-red camera, while the marker means and the pointer means may each carry infra-red reflector means. Alternatively the detector means may comprise an ultrasonic emitter or receiver, whereas the marker means and the pointer means may each carry ultrasonic reflector or emitter means.

Once the data has been input the computer can be used to calculate the pre-operative head position. It may be necessary to make adjustments to the calculated position to correct for deformities which may have developed and/or to correct the anatomical position for the head center with relation to the femur and acetabular geometries.

The overlaying of data relating to the size and shape of the at least one femoral head resurfacing prosthesis on the displayed model may be illustrated in any suitable means.

Data relating to the geometries of more than one femoral head resurfacing prosthesis may be included in the computer. The model of the patient's hip can then be used to simulate the range of motion that should be obtainable using various head sizes and chosen head center positions. Data relating to the geometries of one or more acetabular hip prosthesis may also be included in the data which may be present in the computer such that the display can illustrate the range of motion with various cup prosthesis. This will enable a judgment to be made of how much sacrifice of acetabular bone will be required for the chosen prosthesis.

A combination of parameters obtained during the preoperative scanning, any data input during the procedure and the judgment of the surgeon when viewing the model will enable the optimal patient outcome to be obtained.

The computer can then be used to provide a schematic representation of the femoral head, with the prosthesis in position, joined with the bony neck of the femur. It is at this stage that the size and position of the prosthesis is considered. The system will then be used such that the computer orientates the opening in the prosthesis in correct alignment with the bony neck. The computer of the system can then assist the surgeon to assess, or carries out the assessment, as to whether the resurfacing head prosthesis chosen is large enough to encompass the neck without notching and determine the angle for the cylindrical axis relative to the femoral bone.

It will be understood that it may be necessary to make a compromise on head size and position so that there is sufficient bone support for the head. This can be achieved by the user making adjustments to the model and the computer displaying the results of such adjustments.

The full range of motion of the patient's hip as it would be with a particular prosthesis can then be simulated using the model with the chosen head and orientation. This enables the surgeon to verify that the chosen head and orientation is correct.

To facilitate the simulation of the full range of motion available with a particular femoral head resurfacing prosthesis, the memory may also include information relating to one or more acetabular cup prosthesis.

Once the procedure detailed above has been carried out, the surgeon can utilize the information to position the cutting instruments in the correct position to achieve the desired machining for correct positioning of the femoral head resurfacing prosthesis.

The display means can be a conventional computer screen. The computer may be provided with a keyboard or other suitable input device or may be a touch operated screen.

According to a second aspect of the present invention there is provided a computer program element for locating the correct position for a femoral head resurfacing prosthesis, the computer program element comprising computer code means which when loaded on a computer will be adapted:
(i) to receive data obtained during preoperative scanning of the patient and display a model of a patient's hip joint using said data;
(ii) to hold data relating to the size and shape of at least one femoral head resurfacing prosthesis;
(iii) to determine the pre-operative head center position of the patient's femoral head;
(iv) optionally to receive input from a user to allow for amendment of the determined head center postion;
(v) to overlay the data relating to the at least one hip resurfacing prosthetic component on the model;
(vi) to simulate the range of motion obtainable with the overlaid hip resurfacing prosthetic component;
(vii) to enable steps (v) and (vi) to be repeated as required optionally using different sizes of hip resurfacing prosthetic components at chosen head center position to select an optimum hip resurfacing prosthetic component;
(viii) to simulate joining the selected prosthetic component to the bony neck; and optionally
(ix) to determine whether the size is correct and the angle for the orientation of the cylindrical axis relative to the femoral bone.

The user may use the output from the computer program element to determine whether the size of the component is correct and/or whether the angle for the orientation of the cylindrical axis relative to the femoral bone is correct. However in a preferred arrangement, the computer program element may also be adapted to determine whether the size is correct and whether the angle for the orientation of the cylindrical axis relative to the femoral bone is correct.

The data required to produce the model of the patient's hip can be obtained and input into the computer program element by any suitable means. Examples of suitable means are discussed above in connection with the computer system of the first aspect.

Once the data has been input the computer program element can be used to calculate the pre-operative head position. It may be necessary to make adjustments to the calculated position to correct for deformities which may have developed and/or to correct the anatomical position for the head center with relation to the femur and acetabular geometries.

Data relating to the geometries of more than one femoral head resurfacing prosthesis may be input or provided in the computer program element. The model of the patient's hip can then be used to simulate the range of motion that should be obtainable using various head sizes and chosen head center positions. A judgment can be made of how much sacrifice of acetabular bone will be required for the chosen prosthesis. In one arrangement, data relating to geometries of one or more acetabular cup prosthesis may also be input or provided in the computer program element.

A combination of the measured parameters, the input data and the judgment of the surgeon when viewing the model will enable the optimal patient outcome to be obtained.

The computer program element can then be used to provide a schematic representation of the head ball being joined with the bony neck. It is at this stage that the size and position of the prosthesis is considered. The computer program element will then be used to orientate the opening in the prosthesis in correct alignment with the bony neck. The computer program element can then assess whether the ball chosen is large enough to encompass the neck without notching and determine the angle for the cylindrical axis relative to the femoral bone.

It will be understood that it may be necessary to make a compromise on head size and position so that there is sufficient bone support for the head. This can be achieved by the user making adjustments to the model and the computer program element displaying the results of such adjustments.

The full range of motion can then be simulated using the model with the chosen head and orientation.

Once the steps of the present invention have been carried out, the surgeon can position the cutting instruments in the correct position to achieve the desired machining for correct positioning of the head.

According to a third aspect of the present invention there is provided a computer program comprising the computer program element of the above second aspect.

According to a fourth aspect of the present invention there is provided media including the computer program element of the above second aspect or the computer program of the above third aspect.

According to a fifth aspect of the present invention there is provided a computer programmed with the computer program of the above third aspect

It will therefore be understood that the user may use the output from the computer program element, computer program or computer to determine whether the size of the component is correct and/or whether the angle for the orientation of the cylindrical axis relative to the femoral bone is correct. However in a preferred arrangement, the computer program may also be adapted to determine whether the size is correct and whether the angle for the orientation of the cylindrical axis relative to the femoral bone is correct.

The computer of may be connected to a printer which may be used to print out an operative note. The computer may also be connected to a bar code reader. The bar code reader may be used to input the details of the prostheses used.

The computer program element, computer program or computer of any aspect of the present invention can, if desired, include a feature whereby the surgeon can input desired remarks. Such remarks can be input via a computer keyboard or any other suitable means including voice activated data input. The surgeon can in this way have an automatically produced operative note.

Either at the beginning of the operation or at the end thereof, the computer program element, computer program or computer includes a display screen where administrative data such as the patient's name, the patient's record number, operative date, the name of the surgeon, whether the operation has been upon the left hip or the right hip, and the like, can be entered.

It is also possible for the computer program element, computer program or computer to record at least some of the patient's physiological data, such as blood pressure, heart rate, and the like. Also the details of the anesthetic procedures used could be recorded by the computer program element, computer program or computer.

Although the computer program of the present invention has been described in relation to a hip replacement operation using hip resurfacing technology, the general teachings of the invention can be applied to the any other type of hip prosthesis or indeed to any other orthopaedic or other surgical procedure in which success requires careful alignment of components.

The system, computer program element, computer program, media or computer of the present invention may be used in a method for locating the correct position for a hip resurfacing prosthesis comprising the steps of:
(i) obtaining a model of the patient's hip joint;
(ii) determining the pre-operative head center position of the patient's femoral head;
(iii) optionally amending the determined head center position;
(iv) overlaying the hip resurfacing prosthetic component on the model;
(v) simulating the range of motion obtainable with the overlaid prosthetic components
(vi) repeating steps (iv) and (v) as required using various sizes of hip resurfacing prosthetic components at chosen head center position to select optimum prosthetic component;
(vii) simulating joining the selected prosthetic component to the bony neck; and
(viii) determining whether the size is correct and the angle for the orientation of the cylindrical axis relative to the femoral bone.

The method steps of the present invention will preferably be carried out using computer simulation and navigation techniques.

Whilst the model of the present invention may be taken as a three dimensional model, it will generally be a virtual model displayed using a computer system on a computer screen.

The model of the patient's hip can be input into a computer system by any suitable means. In one arrangement, scanning techniques such as X-Ray, computer aided tomography scanning and the like may be used to take the dimensions to form the model.

In one arrangement a number of landmark points of some critical elements of the bone surface may be plotted at rest and while the patient's leg is moving. This will generally be done at an early stage of joint exposure but prior to the dislocation of the hip joint. Any suitable arrangement of recording the position of these landmark points may be used. Examples of suitable techniques are described in EP1226788. For example, marker means may be attachment to appropriate positions on the bones at and/or near the joint. Pointer means may be provided to touch significant features on the joint. The marker means and the pointer means may all be adapted for operative coupling to a detector means. In a system of this type, the detector means may comprise an infra-red camera, while the marker means and the pointer means may each carry infra-red reflector means. Alternatively the detector means may comprise an ultrasonic emitter or receiver, whereas the marker means and the pointer means may each carry ultrasonic reflector or emitter means.

Once the data has been input the computer can be used to calculate the pre-operative head position. It may be necessary to make adjustments to the calculated position to correct for deformities which may have developed and/or to correct the anatomical position for the head center with relation to the femur and acetabular geometries.

The overlaying of data relating to the size and shape of the at least one femoral head resurfacing prosthesis on the displayed model may be illustrated in any suitable means.

Data relating to the geometries of more than one femoral head resurfacing prosthesis may be included in the computer. The model of the patient's hip can then be used to simulate the range of motion that should be obtainable using various head sizes and chosen head center positions. Data relating to the geometries of one or more acetabular hip prosthesis may also be included in the data which may be present in the computer such that the display can illustrate the range of motion with various cup prosthesis. This will enable a judgment to be made of how much sacrifice of acetabular bone will be required for the chosen prosthesis.

A combination of parameters obtained during the preoperative scanning, any data input during the procedure and the judgment of the surgeon when viewing the model will enable the optimal patient outcome to be obtained.

The computer can then be used to provide a schematic representation of the femoral head, with the prosthesis in position, joined with the bony neck of the femur. It is at this stage that the size and position of the prosthesis is considered. The system will then be used such that the computer orientates the opening in the prosthesis in correct alignment with the bony neck. The computer of the system can then assist the surgeon to assess, or carries out the assessment, as to whether the resurfacing head prosthesis chosen is large enough to encompass the neck without notching and determine the angle for the cylindrical axis relative to the femoral bone.

It will be understood that it may be necessary to make a compromise on head size and position so that there is sufficient bone support for the head. This can be achieved by the user making adjustments to the model and the computer displaying the results of such adjustments.

The full range of motion of the patient's hip as it would be with a particular prosthesis can then be simulated using the model with the chosen head and orientation. This enables the surgeon to verify that the chosen head and orientation is correct.

To facilitate the simulation of the full range of motion available with a particular femoral head resurfacing prosthesis, the memory may also include information relating to one or more acetabular cup prosthesis.

Once the procedure detailed above has been carried out, the surgeon can utilize the information to position the cutting instruments in the correct position to achieve the desired machining for correct positioning of the femoral head resurfacing prosthesis.

## Claims

1. A system for use by a surgeon during implantation of a femoral head resurfacing prosthesis in a patient which will locate the correct position for the femoral head resurfacing prosthesis, the system comprising:
(a) a computer having memory for holding data relating to the size and shape of at least one femoral head resurfacing implant, and data obtained during preoperative scanning of the patient's hip joint;
(b) said computer having a display suitable for displaying a model of the patient's hip joint; and
(c) said computer being programmed to:
(i) determine the pre-operative head center position of the patient's femoral head;
(ii) optionally amend the determined head center position;
(iii) overlay the data relating to the size and shape of the at least one femoral implant on the model;
(iv) simulate the range of motion obtainable with the overlaid prosthetic implant;
(v) repeat steps (iii) and (iv) as required optionally using a different size hip resurfacing prosthetic component at chosen head center position to select optimum femoral implant;
(vi) simulate joining the selected prosthetic component to the bony neck; and optionally
(vii) determine whether the size is correct and the angle for the orientation of the cylindrical axis relative to the femoral bone.

2. A system according to Claim 1 wherein the data required to produce the model of the patient's hip is input into the computer system by a scanning technique.

3. A system according to Claim 1 or 2 wherein the scanning technique is X-Ray or computer aided tomography scanning.

4. A system according to any one of Claims 1 to 3 wherein the computer will additionally calculate the pre-operative head position.

5. A system according to Claim 4 wherein the computer enables the user to make adjustments to the calculated position to correct for deformities and/or to correct the anatomical position for the head center with relation to femoral and acetabular geometries.

6. A system according to Claim 4 wherein the computer makes adjustments to the calculated position to correct for deformities and/or to correct the anatomical position for the head center with relation to femoral and acetabular geometries.

7. A system according to any one of Claims 1 to 6 wherein the computer includes data relating to the geometries of more than one femoral head resurfacing prosthesis.

8. A system according to any one of Claims 1 to 7 wherein the computer includes data relating to acetabular cup prosthesis.

9. A computer program element for locating the correct position for a hip resurfacing prosthesis, the computer program element comprising computer code means which when loaded on a computer will be adapted:
(i) to receive data and display a model of a patients hip joint;
(ii) to determine the pre-operative head center position of the patients femoral head;
(iii) to receive input from a user to allow for optional amending the determined head center postion;
(iv) to overlay the hip resurfacing prosthetic component on the model;
(v) to simulate the range of motion obtainable with the overlaid prosthetic components
(vi) to enable steps (iv) and (v) to be repeated as required using various sizes of hip resurfacing prosthetic components at chosen head center position to select optimum prosthetic component;
(vii) to simulate joining the selected prosthetic component to the bony neck and optionally
(vii) determine whether the size is correct and the angle for the orientation of the cylindrical axis relative to the femoral bone.

10. A computer program element according to Claim 9 wherein data relating to the geometries of more than one femoral head resurfacing prosthesis is included.

11. A computer program according to Claim 9 or 10 wherein data relating to one or more acetabular cup prosthesis are included in the computer program element.

12. A computer program element adapted to provide a schematic representation of the femoral head prosthesis being joined with the bony neck.

13. A computer program comprising the computer program element of any one of Claims 9 to 12.

14. Media including the computer program of Claim 13.

15. A computer programmed with the computer program of Claim 13.
